# EUROPEAN PATENT APPLICATION

(11) **EP 2 399 524 A1**
(43) Date of publication of application: **28.12.2011**
(21) Application number: 10166951.3
(22) Date of filing: 22.06.2010
(51) Int. Cl.: A61B 17/00

(54) **Medical implant and manufacturing method thereof**

(71) Applicant: Occlutech Holding AG, 8201 Schaffhausen (CH)
(72) Inventor: Ottma, Rüdiger, 99441, Grossschwabenhausen (DE); Nielsen, Stevan, 72108, Rottenburg am Neckar (DE)
(74) Representative: KIPA

(57) **Abstract**

A medical implantable occlusion device (100, 200, 300) is disclosed comprising at least one thread (101) and a structural formation (102) thereof having a collapsed and an expanded shape. The formation comprises a first and a second portion (103, 104) and at least one intermediate fixing member (120, 121, 125) attached to the formation between the first and second portion. The at least one intermediate fixing member is arranged to fix the position of the at least one thread in the first portion in relation to the position of the at least one thread in the second portion.

## Description

### Field of the Invention

This invention pertains in general to the field of medical implants, as well as methods for manufacturing such implants. More particularly the invention relates to an intraluminally deliverable occlusion device for selective occlusion of a target site in a body lumen, such as the body's circulatory system.

### Background of the Invention

Various intravascular deliverable devices are used for treating specific conditions via access through body lumina, such as the patient's circulatory system. The target site may for instance be an atrial or ventricular septum having a defective opening to be occluded. In certain circumstances, it may be necessary to occlude a patient's lumen, vessel, chamber, channel, hole, shunt, opening or cavity for treatment.

An occlusion device is disclosed in WO 99/39646 for occluding an abnormal opening. It is disclosed a device of a continuous tubular metal fabric which includes two outer occluding portions, and a resilient central, spring-like interconnecting member. The ends of the metal fabric are welded or clamped together with a clamp to avoid fraying and unraveling of the braid. The clamps tying together the wire strands at the ends also serve to connect the device to a delivery system.

An issue with prior art is insufficient clamping function between outer occluding portions due to the resiliency between the outer portions. This may lead to dislocation of the device with severe health risk to the patient. This may be particularly critical when larger openings are to be occluded requiring larger retaining members on either side of the defect septum, and thereby requiring a larger clamping force not to loose contact with the septum wall at the outer portions of the retaining members. Thus, there is a need for an implantable occluder that has improved retaining or clamping function, avoiding undesired dislocation in particular at large shunts or openings.

Another problem with prior art is the limited flexibility when connecting the occluding device to a delivery device. For example, the construction of prior art occluders inherently limits the possibilities for applying various connections to a delivery device while maintaining a compact occluder. A compact occluder is essential e.g. for delivery trough small catheters, and for reaching sites in the body of complex anatomy. Thus, there is a need for a compact and low-profile implantable occluder providing increased flexibility in choice of connection to a delivery device.

Also, prior art constructions have bulky connections between occluder and delivery device inherent to the design of the occluder, which prevents structural flexibility when delivering the occluder. A potential high flexibility between occluder and delivery device is necessary for safe delivery of the occluder, e.g. in order to be able to determine if the occluder fits perfectly. Thus, there is a need for an implantable occluder with increased structural flexibility between an occluder and delivery device.

In context of the aforementioned disadvantages, prior art such as WO 99/39646 has clamps arranged at the ends of the occluding portions, necessary to prevent fraying or unraveling of the braiding. This increases the bulk of the occluder, which is an even larger problem for larger occluders as there will be more or larger ends to clamp. Further, the construction of the connection between occluder and delivery device is dictated by the clamp at the ends, which is a major disadvantage if the goal is to achieve a compact occluder. Moreover, clamps might loosen from the structure of the occluder in an undesired way when implanted in the body.

Also, an issue with prior art is the insufficient flexibility of the occluding portions.

The above problems may have dire consequences for the patient and the health care system. Patient risk is increased.

Hence, an improved implant would be advantageous and in particular allowing for increased flexibility, cost-effectiveness, and/or patient safety would be advantageous. Also, and a method for manufacturing such medical implant would be advantageous.

### Summary of the Invention

Accordingly, embodiments of the present invention preferably seek to mitigate, alleviate or eliminate one or more deficiencies, disadvantages or issues in the art, such as the above-identified, singly or in any combination by providing a device and a method according to the appended patent claims.

Embodiments of the present invention may be well suited for the selective occlusion of a vessel, lumen, channel, hole, cavity, shunt, opening or the like. Examples, without limitations, are a vessel, lumen, channel, or hole through which blood flows from one vessel to another vessel such as an Atrial Septal Defect (herein after ASD) or a Ventricular Septal Defect (herein after VSD). Other examples could be an Arterial Venous Fistula (AVF), Arterial Venous Malformation (AVM), a Patent Foramen Ovale (PFO), Para-Valvular Leak (PVL), or Patent Ductus Arteriosus.

According to a first aspect of the invention a medical implantable occlusion device is provided comprising at least one thread and a structural formation thereof having a collapsed and an expanded shape. The structural formation comprises a first and a second portion, a longitudinal axis extending between the first and a second portion, and at least one intermediate fixing member attached to the formation between the first and second portion. The at least one intermediate fixing member is arranged to fix the position of the at least one thread in the first portion in relation to the position of the at least one thread in the second portion.

According to a second aspect of the invention a method of manufacturing a medical implantable occlusion device having a formation of at least one thread and having an expanded and a collapsed shape is provided. The method comprises forming a first portion of the structural formation, forming a second portion of the structural formation, attaching at least one intermediate fixing member to the structural formation between the first and second portion, wherein the at least one intermediate fixing member is arranged to fix the position of the at least one thread in the first portion in relation to the position of the at least one thread in the second portion.

According to a third aspect of the invention a method of occluding a shunt in a body lumen is provided. The method comprises providing a device according to the first aspect of the invention, inserting the device in a collapsed state into the shunt, expanding and releasing the device in the shunt, thus anchoring the device in the shunt for occluding the latter by the device.

Further embodiments of the invention are defined in the dependent claims, wherein features for the second and subsequent aspects of the invention are as for the first aspect mutatis mutandis.

Some embodiments of the invention provide for an implantable occluder that has improved clamping function when implanted.

Some embodiments of the invention also provide for a compact and low-profile implantable occluder providing increased flexibility in choice of connection to a delivery device.

Some embodiments of the invention also provide for an implantable occluder with increased structural flexibility between occluder and delivery device.

Some embodiments of the invention provide for a medical implant that can be safely delivered to a treatment site in a patient.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which embodiments of the invention are capable of will be apparent and elucidated from the following description of embodiments of the present invention, reference being made to the accompanying drawings, in which
Fig. 1 is an illustration of a medical implantable occlusion device according to an embodiment of the invention;
Fig. 2 is an illustration of a medical implantable occlusion device according to an embodiment of the invention;
Fig. 3 is an illustration of a medical implantable occlusion device according to an embodiment of the invention;
Figs. 4a-b are illustrations of a medical implantable occlusion device according to an embodiment of the invention during a manufacturing step;
Fig. 5 is a perspective view of a medical implantable occlusion device according to Fig. 1.
Fig. 6 is a schematic illustration of a detail of the medical implantable occlusion device according to an embodiment of the invention;
Fig. 7 is a schematic illustration of a detail of the medical implantable occlusion device according to an embodiment of the invention;
Fig. 8 is a schematic illustration the medical implantable occlusion device according to an embodiment of the invention when occluding a shunt in a body;
Fig. 9 is a flow chart illustrating a method of manufacturing of a medical implantable occlusion device according to an embodiment of the invention; and
Fig. 10 is a flow chart illustrating a method of occluding a shunt or opening in a body lumen with a medical implantable occlusion device according to an embodiment of the invention.

### Description of embodiments

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

The following description focuses on an embodiment of the present invention applicable to a PFO occluder. However, it will be appreciated that the invention is not limited to this application but may be applied to many other medical implantable devices, including for example filters, stents, Left Atrial Appendage (LAA) occluders, aneurysm treatment devices, grafts, etc.

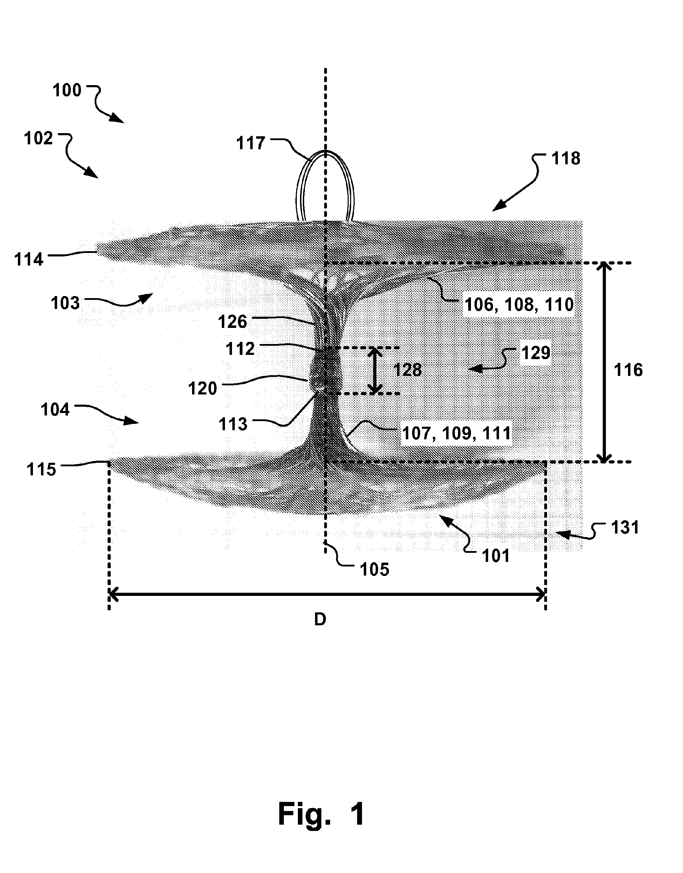

Fig. 1 shows a medical implantable occlusion device 100 according to an embodiment of the invention. The device 100 comprises at least one thread 101 and a structural formation 102 thereof having a collapsed and an expanded shape. The formation 102 comprises a first and a second portion 103, 104, where a longitudinal axis 105 extends between the first and a second portion 103, 104. In Fig. 1 the device 100 is in its expanded shape. The first and second portion 103, 104, are flexible and may be extended in opposite directions along the longitudinal axis 105, whereby a diameter (D) of the first or second portion 103, 104, is reduced such that the device 100 may be accommodated in a delivery catheter, i.e. in the collapsed shape of the device 100. The device 100 may be self-expandable and thereby change shape from the collapsed shape to the expanded shape upon withdrawal from a delivery catheter. During delivery through a catheter the longitudinal axis 105 of the device 100 may be substantially parallel to a longitudinal axis of the catheter.

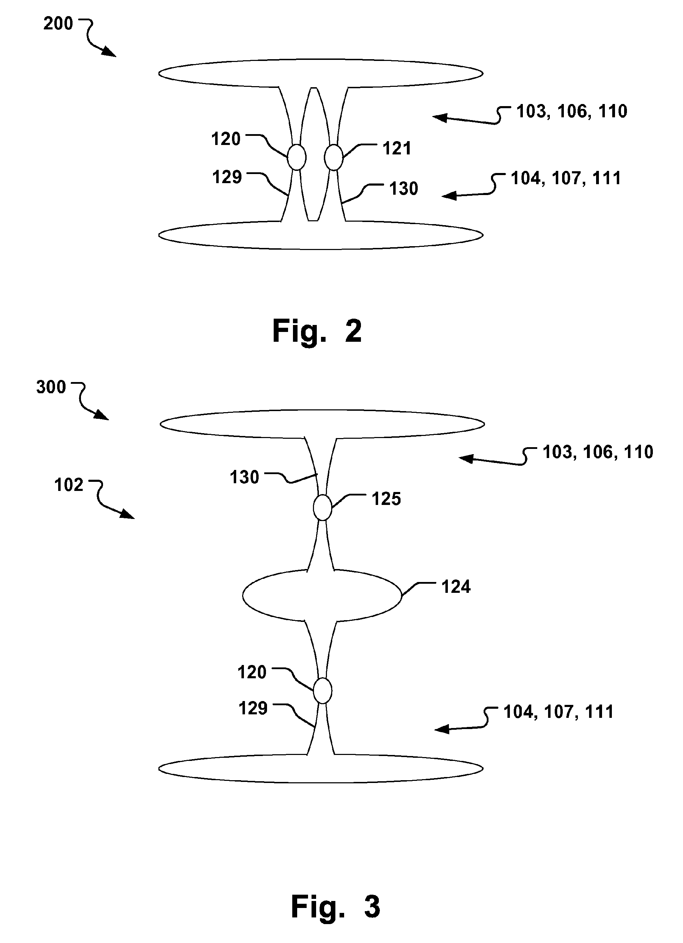

The device 100, 200, 300, comprises at least one intermediate fixing member 120, 121, 125, also shown in the embodiments in Fig. 2 and Fig. 3. The intermediate fixing member 120, 121, 125, is attached to the formation 102 between the first and second portion 103, 104. The intermediate fixing member 120, 121, 125, is arranged to fix the position of the at least one thread in the first portion 103 in relation to the position of the at least one thread in the second portion 104. An increased clamping force between the first and second portion 103, 104, when implanted in a shunt or opening, is thereby achieved as unwanted movement of the threads is prevented. In particular, the at least one thread in the first and second portions 103, 104, adjacent to the intermediate fixing member 120, 121, 125, are substantially prevented from relative movement with respect to each other due to the fixation of the thread to the intermediate fixing member 120, 121, 125. This is particularly advantageous when having large diameter openings or shunts as described further below.

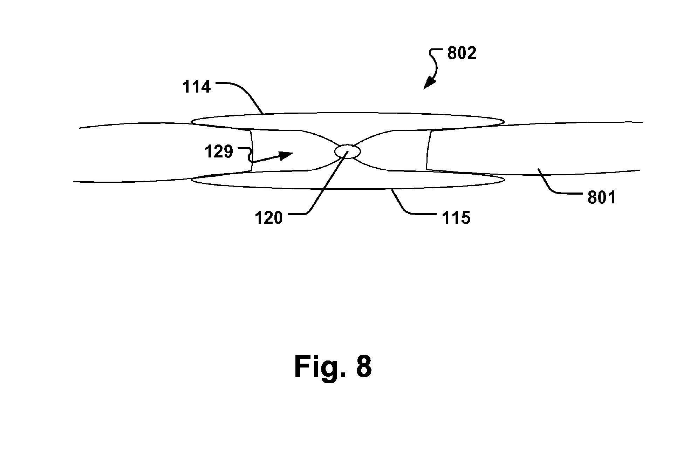

The formation 102 has expanded diameter portions 114, 115, and at least one reduced diameter portion 129, 130, also shown in Fig. 2 and Fig. 3, between the expanded diameter portions 114, 115. The intermediate fixing member 120, 121, 125, is attached to the reduced diameter portion 129, 130. The at least one thread of the formation 102 in the reduced diameter portion 129, 130, is substantially fixed in position as the expanded diameter portions 114, 115, are moved in relation to each other, e.g. in opposite directions along the longitudinal axis 105. Thereby a more controllable and/or increased clamping force between expanded diameter portions 114, 115, may be provided when the device 100, 200, 300, is implanted e.g. in a defect septum 801 with expanded diameter portions 114, 115, on opposite sides of the septum 801, as illustrated in Fig. 8.

The device 100, 200, 300, may thereby have a better hold at the implanted site.

The expanded diameter portions 114, 115, are sufficiently flexible to follow the surface of the septum's surface at their perimeter. As discussed above these portions are flexible for allowing the device 100, 200, 300, to collapse into the collapsed shape during delivery trough a catheter (not shown), and to expand to the expanded shape at the implant site in the body. As only the threads in the reduced diameter portion 129 may be fixed to the intermediate fixing member 120, 121, 125, the expanded diameter portions 114, 115, are flexible to adapt to different wall thicknesses of the septum 801. An optimal retaining or clamping force of the expanded diameter portions is provided from the opposite sides of the septal wall surrounding the opening to be occluded. This advantageous retaining is provided although there might be only a small overlap at the perimeter of expanded diameter portions in relation to the diameter of the opening, while dislocation is effectively prevented upon implantation. The device may then integrate over time with the surrounding tissue at the shunt or opening, e.g. by endotheliazation processes.

It is also advantageous that in this manner the amount of implanted material of the device is reduced thanks to the intermediate fixing member 120, 121, 125, compared to devices which fill the entire shunt or opening with substantially their intermediate portion.

When having to occlude non-circular or asymmetric shunts or openings it is also advantages to be able to provide a relatively small waist of the intermediate portion in relation to the overall diameter of the expanded diameter portions, as provided by the intermediate fixing member 120, 121, 125. The device may thus be more easily implanted in such irregular shunts or openings as the intermediate portion takes little space, and improves the retaining as described above.

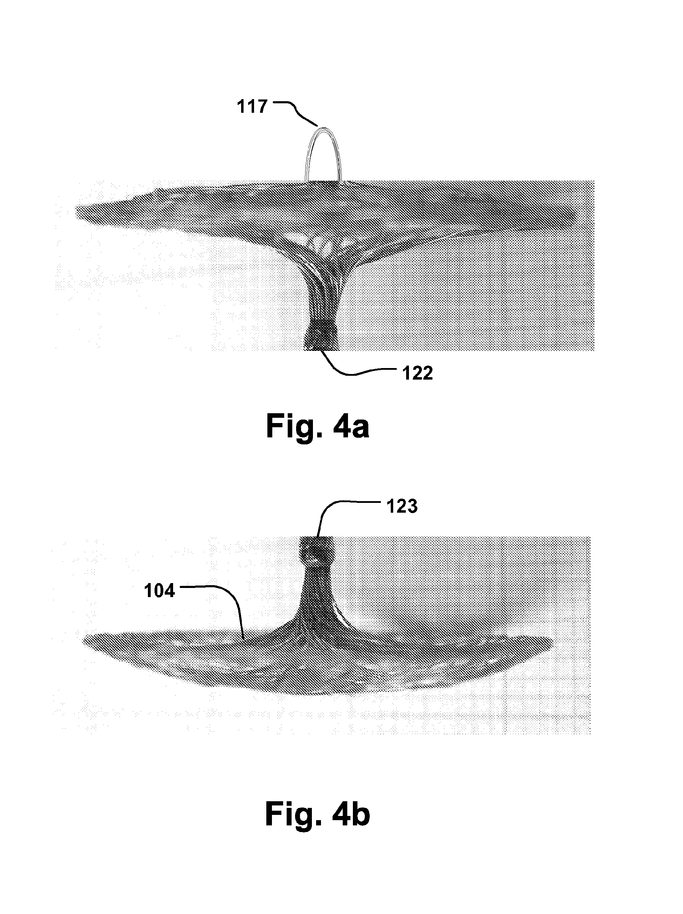

The first portion 103 may comprise at least a first thread 106, and the second portion 104 may comprise at least a second thread 107. Hence, the first thread 106 may not be part of the second portion 104, and the second thread 107 may not be part of the first portion 103. Hence, the first and second portions 103, 104, may be isolated parts prior to being joined as a single formation 102 by the intermediate fixing member 120, 121, 125, as described in relation to Figs. 4a-b.

A multiple of formations of the device 100, 200, 300, may thereby be achieved with an efficient modular manufacturing. Each of the first and second portions 103, 104, may comprise returning loops of each of the threads 106, 107, respectively, where opposite ends of the threads are fixed to the intermediate fixing member 120, 121, 125.

The first portion 103 may accordingly comprise a first thread 106 having one ore more loops forming the first portion 103, and with opposite ends of the first thread 106 fixed to the intermediate fixing member 120, 121, 125, and vice versa for the second portion 104. The ends of the first and second thread 106, 107, are collected at the reduced diameter portion 129, and fixed to the intermediate fixing member 120, 121, 125. The expanded diameter portions 114, 115, have thereby no ends of the first and second thread 106, 107, that needs to be fixed at a proximal end 118, or a distal end 131 of the device 100, 200, 300. A compact device 100, 200, 300, is thereby provided.

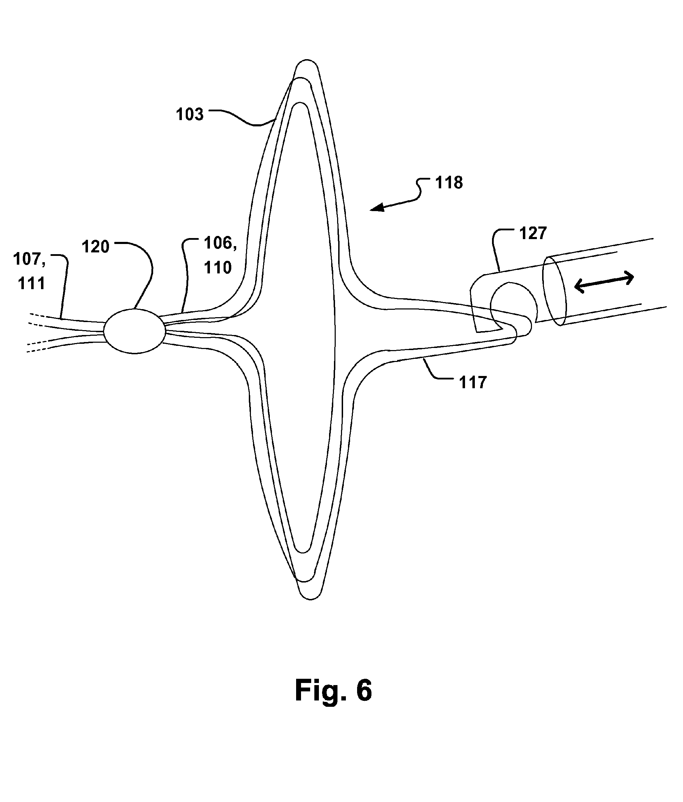

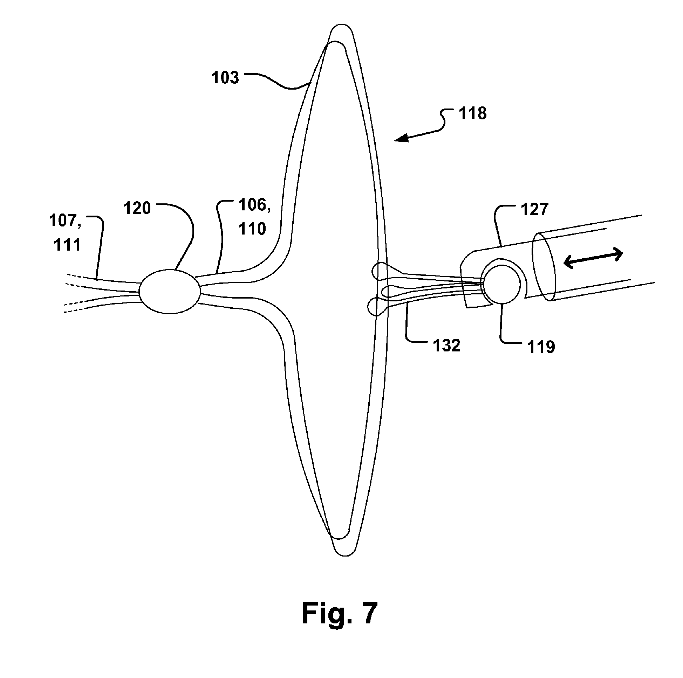

As the proximal and distal end 118, 131, has no ends of the threads 106, 107, of the formation 102 that needs to be fixed, a connecting member 117, 119, may be provided at the proximal and/or distal end 118, 131, having a compact and constant shape independent of the size and/or the amount of threads of the formation 102, and without interference from the ends of the threads 106, 107. The intermediate fixing members 120, 121 125, thereby fulfills the roles of providing stronger clamping between the expanded diameter portions 114, 115, and providing a more compact device 100, 200, 300. The connecting member 117, 119, is discussed in more detail below in relation to Fig. 6 and Fig. 7.

The formation 102 may be comprised of a braiding of threads. The formation 102 may be comprised of at least one braiding, for example, the first portion 103 is comprised of a first braiding and the second portion 103 is comprised of a second braiding. The formation 102 or braiding may be comprised of a bio-compatible material, such as Nitinol. The formation may be made of metal threads. In other embodiments, the formation may be made of a shape memory polymer. The formation or braiding may be heat-treated to set the desired shape in the collapsed shape and/or expanded shape. The braiding may be provided in the type of braiding disclosed in unpublished European patent application EP10163680 of the same applicant, which is incorporated herein by reference in its entirety for all purposes. Such braiding may increase the flexibility of the formation 102.

The intermediate fixing member 120, 121, 125, may comprise in certain embodiments a weld, solder, clamp, a fixing glue, combinations thereof, or any other means for fixing the position of the threads in the reduced diameter portion 129 in relation to each other. Hence, no relative movement may occur between these threads and the intermediate fixing member 120, 121, 125. The intermediate fixing member 120, 121, 125, may thus be substantially inflexible for preventing movement of the at least one thread in the first portion 103 in relation to the position of the at least one thread in the second portion 104. The movement may be prevented in directions such as parallel to the longitudinal axis 105, perpendicular to the longitudinal axis 105, or in any direction. Movement of the threads in the reduced diameter portion 129, 130, is prevented upon displacement of the flexible expanded diameter portions 114, 115, in relation to each other.

Fig. 3 shows a device 300 where the formation 102 comprises a plurality of portions 103, 104, 124, and a plurality of intermediate fixing members 120, 125, attached to the formation 102 between each of the plurality of portions 103, 104, 124. The intermediate fixing members 120, 125, fix the position of the threads between the portions 103, 104, 124. In Fig. 3 the portions 103, 104, 124, are connected in series. More portions may be connected in series or in other configurations in order to customise the device 300 for the desired application. This may in particular be advantageous in order to further increase the clamping or retaining effect of the device. The clamping or retaining effect may be adapted to patient specific anatomy by tailoring the structure of the device to the patient specific anatomical defect, shunt, opening, etc. to be reliably occluded by the device.

The plurality of intermediate fixing members may also be arranged asymmetrically over the diameter of the device in order to adapt the occlusion device to irregular defects, shunts, or openings to be occluded. Upon delivery and expansion of the device, the device will self-rotate into place with the intermediate portion having a reduced diameter and being defined in shape by the asymmetric arrangement of the plurality of intermediate fixing members. In this manner, irregular defects may be occluded advantageously and without the need for any means during delivery ensuring the rotational orientation of the device in relation to the irregular defect.

The first portion 103 may comprise a first plurality of returning loops 110 of a first plurality of threads and the second portion 104 may comprise a second plurality of returning loops 111 of a second plurality of threads. The intermediate fixing members 120, 121, 125, may accordingly be fixed to a plurality of opposite ends of each of the returning loops 110, 111. As shown in Fig. 1, the at least one intermediate fixing member 120, 121, 125, is a first intermediate fixing member 120 having a first connecting end 112 and a second connecting end 113. Opposite ends of the first plurality of returning loops 110 are fixed to the first connecting end 112, and opposite ends of the second plurality of returning loops 111 are fixed to the second connecting end 113. The opposite ends the first plurality of returning loops 110 form a first bundle of strands 126 substantially arranged in parallel. Likewise, the opposite ends the second plurality of returning loops 111 form another bundle of strands arranged in parallel. The parallel arrangement of the bundle of strands 126 may increase the rigidity of the reduced diameter portion 129, and thereby increase the clamping force of the first expanded diameter portion 114 towards the second expanded diameter portion 115. This advantageously further increases the clamping or retaining effect of the device, as desired.

In Fig. 1 the first and second connecting ends 112, 113, are arranged on opposite sides of the intermediate fixing member 120 along the longitudinal axis 105. The first and second connecting ends 112, 113, may be arranged in varying angle to each other whereby the expanded diameter portions 114, 115, may have an angular displacement to each other for customization to certain applications or anatomies. The device 100 has the first bundle of strands 126 arranged substantially parallel with the longitudinal axis 105. Thereby the threads of the bundle of strands 126 is substantially prevented from moving in relation to each other as the expanded diameter portions 114, 115, are moved in relation to each other along the longitudinal axis 105.

As shown in Fig. 3 the device 300 comprises a plurality of intermediate fixing members 120, 121, each fixing one or more ends of the first plurality of returning loops 110 of the first portion 103 to one or more ends of the second plurality of returning loops 111 of the second portion 104. In this case, the returning loops 110, 111, are grouped in two bundle of strands for each portion 103, 104, and fixed to the intermediate fixing members 120, 121. The device 300 may comprise more groups of strands bundled together, each joined to a corresponding intermediate fixing member. Ultimately, the ends of each of the returning loops 110 in the first portion 103 may be fixed to the ends of each of the returning loops 111 in the second portion 111, by a corresponding plurality of intermediate fixing members.

At least one of the first and second portions comprises a resilient expanded diameter portion 114, 115. In Fig. 1 the separation distance 116 between the first and second expanded diameter portions 114, 115, is less than a thickness of a patient's atrial septum. An increased clamping force between the expanded diameter portions 114, 115 may thus be achieved. The length of the reduced diameter portion 129 may correspond substantially to the separation distance 116 between the first and second expanded diameter portions 114, 115. The at least one thread of the expanded diameter portion 114, 115, may be gradually curved inwards to the reduced diameter portion 129 from the expanded diameter portions 114, 115.

The length 128 of the intermediate fixing member 120 in relation to the length of the reduced diameter portion 129 may be varied for adjustment of the clamping force between the expanded diameter portions 114, 115. An intermediate fixing member 120 extending only across a small fraction of the length of the reduced diameter portion 129 may provide a more resilient reduced diameter portion 129, and a reduced clamping force, if the properties of the expanded diameter portions 114, 115, are kept constant.

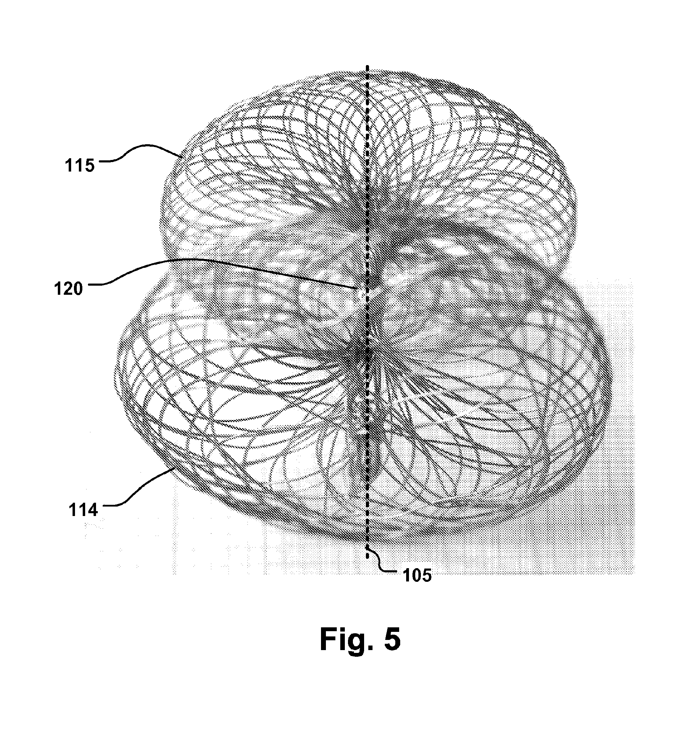

Fig. 5 shows a perspective view of the device 100. The expanded diameter portions 114, 115, are circular and substantially of the same diameter. The diameters may be different however, and further the expanded diameter portion 114, 115, may be eccentric and not symmetrically arranged along the longitudinal axis 105. Those skilled in the art will appreciate that the device 10 is sized in proportion to the shunt to be occluded. The diameter (D) of the expanded diameter portions 114, 115, may be varied as desired for differently sized openings in the septal wall 801.

Fig. 6 and Fig. 7 show a device 100, 200, 300, with a connecting member 117, 119, at the proximal end 118 of the first portion 113. The second portion 104 of the device 100, 200, 300, having a second thread 107 is not shown for clearer presentation. The exemplary delivery device 127 comprises a hook for grasping the connecting member 117, 119, and is subsequently pulled inwards to a lumen for securing attachment between to the device 100, 200, 300 during delivery. Unlocking of the secured attachment may be made when the medical device is delivered to the desired location in the body to be occluded.

In Fig. 6 the connecting member 117 comprises at least one loop strand 117 for engaging with a delivery device 127. The number of loops strands 117 may vary. Fewer loop strands 117 may provide a more compact connecting member 117, and increased flexibility between the proximal end 118 and the delivery device 127. More loops strands 117, and/or thicker loops strands 127 may provide a more rigid connection to the delivery device 127. The at least one loop strand 117 may be integrally formed by the at least one thread in the first portion 103. Thus, the thread 106 or returning loops 110 are looped outwards to form the loop strands 117. As pointed out, the appropriate number of loop strands 117 may be chosen, independently of the size, shape or configuration of the first portion 103. The same delivery device 127 may thus be used for manipulation and delivery of different devices 100, 200, 300, of varying sizes. In addition, the loop strands 117 provide for a recapture of the occlusion device by the delivery device, once released. The loop strands 117 may be caught in the body by the hook of the delivery device. Thus for instance explantation of the device is facilitated.

A delivery device of the type of device 127 is described in unpublished international application PCT/EP2010/056140 of the same applicant as the present application, which hereby is incorporated herein in its entirety for all purposes.

Fig. 7 shows a connecting member comprising an attachment unit, such as the spherical portion 119, for engaging with a delivery device 127. The spherical portion 119 is coupled to the proximal end 118 via loops 132. Thus, the spherical portion 119 may not be integral with the thread 106 forming the first portion 103. A greater flexibility in customizing the spherical portion 119 to fit a particular delivery device 127 may thus be obtained, independent of the structural formation of the first portion 103. The loops 132 are securely held by the spherical portion 119. The loops 132 may provide for folding the unit of the loops 132 and the spherical attachment unit 119 in a direction perpendicular to the longitudinal axis of the device.

Due to the returning loops 110, the connecting member 117, 119, may be freely adapted and of compact size, as the ends of the thread 106 are fixed to the intermediate fixing member 120, 121, 125, at the opposite side of the first portion 103 from the connecting member 117, 119.

The connecting member 117, 119 may be arranged and shaped to have a flexibility substantially perpendicular to the longitudinal axis of the occlusion device. In this manner, the connecting member 117, 119, which is illustrated in the Figs. in a state protruding from an end of the device, may have a second state where it is substantially not protruding from the end, being flat folded back to the end surface of the occlusion device. This is particularly advantageous in applications where it is desired to have a substantially flat end of the occlusion device at the delivery end when it is implanted.

During delivery, the delivery device 127 may be attached to the loops of the connecting member 117 or to the attachment unit, such as to the spherical unit 119, against the flexibility of the connecting member 117, 119. Thus, during delivery, the connecting member 117, 119 may be arranged substantially in line and along the longitudinal axis. This is advantageous for catheter based delivery allowing for small diameter catheters, while having no protruding ends at the device after delivery. The flexibility of the connecting member 117, 119 may be provided to fold the latter with its length in a direction perpendicular to the longitudinal axis. Alternatively, or in addition, the structure may flatten along the longitudinal axis, like rings of an onion, and be resiliently drawn away from the device end for protruding during delivery, as illustrated in the Figs. when releasing the delivery device, the connecting member 117, 119 returns to the substantially flat arrangement, leaving substantially no protruding elements from the end of the occlusion device.

In certain embodiments, said at least one intermediate fixing member is arranged closer to said first portion than said second portion (not shown in the Figures). This asymmetrical arrangement in the longitudinal direction of the device may be particularly advantageous in certain anatomical situations, where it is desired to apply the resilient force more on one side than the other. In embodiments having a plurality of intermediate members, a defined region may be provided with this effective patient adaptation. Asymetric anatomical defects to be closed by the device may also include an asymmetric thickness of the tissue in which the device is anchored at the perimeter. Furthermore, the length of the intermediate portion may be chosen differently for different intermediate portions of a plurality of intermediate portions. Thus an advantageous tension and reliable retention of the device is provideable. In combination with the aforementioned auto rotation into place upon delivery, extremely effective devices are provided.

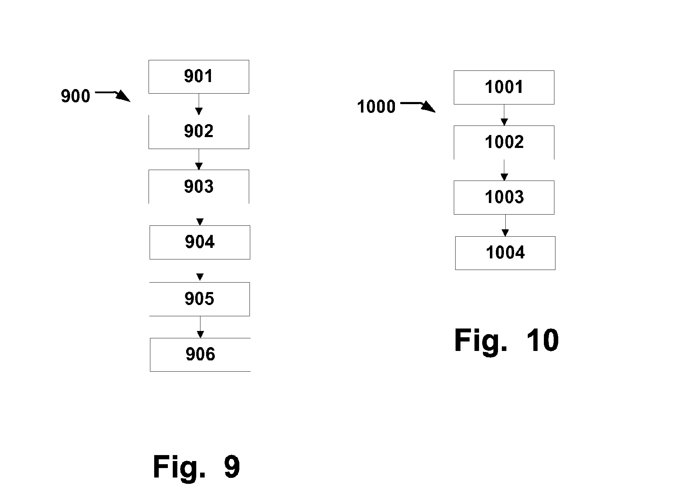

Fig. 9 illustrates a method 900 of manufacturing a medical implantable occlusion device 100, 200, 300, having a structural formation 102 of at least one thread 101 and having an expanded and a collapsed shape. The method 900 comprises a production process for the device including forming 901 a first portion 103 of the structural formation 102, forming 902 a second portion 104 of the structural formation 102. The method comprises further attaching 903 at least one intermediate fixing member 120, 121, 125, to the structural formation 102 between the first and second portion 103, 104. As previously described the at least one intermediate fixing member is arranged to fix the position of the at least one thread in the first portion 103 in relation to the position of the at least one thread in the second portion 104. The device 100, 200, 300, may thus be formed prior to attaching the at least one intermediate fixing member 120, 121, 125.

Figs. 4a-b illustrates the method 900 where a first fixation member 122 is attached 904 to the ends of the first plurality of threads of the first portion 103. A second fixation member 123 is attached 905 to the ends of the second plurality of threads of the second portion 104. The first and second fixation members 122, 123, are then attached 906 to each other to form the intermediate fixing member 120, 121, 125. Thus, the intermediate fixing member 120, 121, 125, may comprise of at least two fixation members 122, 123. The formation 102 is consequently provided by fixing the two separate first and second portions 103, 104 to each other.

The shape of the first and second portions 103, 104, may be formed by heat-treatment after fixing the first and second portions 103, 104, to each other. The attachment of the first and second fixation members 122, 123, may be omitted, and the intermediate fixing member 120, 121, 125, may be attached directly to the ends of the first and second threads 106, 107, of the first and second portions 103, 104, simultaneously or in sequence.

Fig. 10 is a flow chart illustrating an exemplary method 1000 of using the aforementioned described occlusion device. The device is used for occluding a shunt 802 in a body lumen with a medical implantable occlusion device according 100, 200, 300, see further Fig. 8. The method 1000 comprises providing 1001 a device 100, 200, 300, inserting 1002 the device 100, 200, 300, in a collapsed state into the shunt 802, expanding 1003 and releasing the device 100, 200, 300, in the shunt 802, thus anchoring 1004 the device 100, 200, 300, in the shunt 802 for occluding the latter by the device 100, 200, 300.

The shunt may be a Patent Ductus Arteriosus (PDA), Arterial Venous Fistula (AVF), Arterial Venous Malformation (AVM), or a Para-Valvular Leak (PVL).

Thus, in the first portion 103 is retainingly arranged on a first side of said shunt or opening and said second portion 104 is retainingly arranged on a second side of said shunt or opening and wherein the longitudinal axis 105 of said device is extending between said first and second portion substantially along said shunt and/or perpendicular to said opening, and wherein said at least one intermediate fixing member 120, 121, 125 is arranged in said shunt or opening between said opposed first and second side.

A particular advantageous retaining of the device is obtained by the method when at least one of said first and second portions comprises a resilient expanded diameter portion 114, 115, and the separation distance 116 between said first and second expanded diameter portion is less than a thickness of a patient's septum in which said shunt or opening is located, and when furthermore said at least one intermediate fixing member is attached to said formation at a reduced diameter portion thereof 129, 130 arranged in said shunt or opening.

The intermediate fixing member is improving a resilient hold of said device in said shunt or opening upon delivery before an integration with tissue surrounding said shunt or opening of said device occurs, such as by endotheliazation of said device.

The occlusion device may be made of a biodegradable material, which is chosen to controllably degrade when the device has securely occluded the shunt, opening, etc.

The present invention has been described above with reference to specific embodiments. However, other embodiments than the above described are equally possible within the scope of the invention. The different features and steps of the invention may be combined in other combinations than those described. The scope of the invention is only limited by the appended patent claims. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used.

## Claims

1. A medical implantable occlusion device (100, 200, 300, 400, 500) comprising
at least one thread (101) and a structural formation (102) thereof having a collapsed and an expanded shape, said formation comprises a first and a second portion (103, 104),
a longitudinal axis (105) extending between said first and second portion,
at least one intermediate fixing member (120, 121, 125) attached to said formation between said first and second portion, wherein said at least one intermediate fixing member is arranged to fix the position of said at least one thread in the first portion (103) in relation to the position of said at least one thread in the second portion (104).

2. Occlusion device according to claim 1, wherein said first portion comprises at least a first thread (106), and said second portion comprises at least a second thread (107), wherein each of said first and second portion comprises returning loops of each of said threads respectively, whereby opposite ends of said threads are fixed to said at least one intermediate fixing member.

3. Occlusion device according to claim 1 or 2, wherein said formation comprises at least one fabric, such as a braiding, of said at least one thread (101), and/or wherein said at least one intermediate fixing member comprises a weld, and/or wherein said wherein said at least one intermediate fixing member is arranged closer to said first portion than said second portion.

4. Occlusion device according to any of claims 1-3,
wherein said formation comprises a plurality of portions (103, 104, 124) and a plurality of intermediate fixing members (120, 125) whereof at least one is attached to said formation between each of said plurality of portions.

5. Occlusion device according to any of claims 1-4,
wherein said first portion comprises a first plurality of returning loops (110) of a first plurality of threads and said second portion comprises a second plurality of returning loops (111) of a second plurality of threads.

6. Occlusion device according to claim 5, wherein said at least one intermediate fixing member comprises a first intermediate fixing member (120) having a first connecting end (112) and a second connecting end (113), wherein opposite ends of said first plurality of returning loops are fixed to said first connecting end, and opposite ends of said second plurality of returning loops are fixed to said second connecting end, and/or
wherein opposite ends of at least said first plurality of returning loops form a first bundle of strands (126) substantially arranged in parallel, and/or
wherein said at least one intermediate fixing member comprises a plurality of intermediate fixing members (120, 121) each fixing one or more ends of said first plurality of returning loops to one or more ends of said second plurality of returning loops.

7. Occlusion device according to claim 6, wherein said first and second connecting ends are arranged on opposite sides of said first intermediate fixing member along said longitudinal axis, and/or wherein said first bundle of strands are arranged substantially parallel with said longitudinal axis.

8. Occlusion device according to any of claims 1-7,
wherein said intermediate fixing member is substantially inflexible for preventing movement of said at least one thread in said first portion along said longitudinal axis in relation to the position of said at least one thread in the second portion.

9. Occlusion device according to any of claims 1-8,
wherein at least one of said first and second portions comprise a resilient expanded diameter portion (114, 115), and wherein a separation distance (116) between said first and second expanded diameter portion is less than a thickness of a patient's septum, and/or wherein said at least one intermediate fixing member is attached to said formation at a reduced diameter portion thereof (129, 130).

10. Occlusion device according to any of claims 1-9,
wherein one of said first and second portions comprises a connecting member (117, 119) at a proximal end (118).

11. Occlusion device according to claim 10, wherein said connecting member comprises at least one loop strand (117) for engaging with a delivery device (127), and/or wherein said connecting member comprises a spherical portion (119) for engaging with a delivery device, and/or wherein said connecting member (117, 119) is arranged and shaped to have a flexibility substantially perpendicular to the longitudinal axis of the occlusion device.

12. Occlusion device according to claim 11, wherein said at least one loop strand is integrally formed by said at least one thread, or wherein said spherical portion (119) is a clot of material at end portions of second threads looped over portions of said at least one thread such that said second threads are secured to said device, and wherein said second threads preferably have a larger thread diameter than a thread diameter of said at least one thread.

13. Method (900) of manufacturing a medical implantable occlusion device having a structural formation of at least one thread and having an expanded and a collapsed shape, such as a device of any of claims 1-12, said method comprising
forming (901) a first portion (103) of said structural formation,
forming (902) a second portion (104) of said structural formation,
attaching (903) at least one intermediate fixing member (120, 121, 125) to said structural formation between said first and second portion, wherein said at least one intermediate fixing member is arranged to fix the position of said at least one thread in the first portion in relation to the position of said at least one thread in the second portion.

14. Method of manufacturing a device according to claim 13, wherein attaching said at least one intermediate fixing member comprises
attaching (904) the ends of a first plurality of threads of said first portion to a first fixation member (122),
attaching (905) the ends of a second plurality of threads of said second portion to a second fixation member (123),
attaching (906) said first and second fixation members so as to form said at least one intermediate fixing member.

15. Method of manufacturing a device according to claim 13 or 14, wherein the steps of forming said first and second portions are performed after attaching said at least one intermediate fixing member to said formation.
